# EUROPÄISCHE PATENTSCHRIFT

(11) **EP 4 072 606 B1**
(45) Veröffentlichungstag und Bekanntmachung des Hinweises auf die Patenterteilung: **17.07.2024**
(21) Anmeldenummer: 20823778.4
(22) Anmeldetag: 09.12.2020
(51) Int. Cl.: A61L 27/06, A61L 27/04, A61L 27/08, A61L 27/10, A61L 27/30, A61L 27/32, A61L 27/54

(54) **IMPLANTAT, IMPLANTATKOMPONENTE UND VERFAHREN ZU DEREN HERSTELLUNG**
IMPLANT, IMPLANT COMPONENT AND METHOD FOR THE PRODUCTION THEREOF
IMPLANT, COMPOSANT D'IMPLANT ET MÉTHODE POUR LEUR PRODUCTION

(30) Priorität: 11.12.2019 DE 102019219355
(43) Veröffentlichungstag der Anmeldung: 19.10.2022
(73) Patentinhaber: Fraunhofer-Gesellschaft zur Förderung der angewandten Forschung e.V., 80686 München (DE)
(72) Erfinder: HOLECZEK, Harald, 79801 Hohentengen am Hochrhein (DE)
(74) Vertreter: Pfenning, Meinig & Partner mbB
(86) Internationale Anmeldenummer: PCT/EP2020/085181
(87) Internationale Veröffentlichungsnummer: WO 2021/116143

(56) Entgegenhaltungen:
- EP-A1- 1 975 277
- DE-A1- 10 158 302
- DE-A1- 102013 201 885
- US-A1- 2002 143 398

## Beschreibung

Es wird ein Implantat und/oder eine Implantatkomponente bereitgestellt, das/die einen Grundkörper aufweist, der zumindest an einer Oberfläche ein elektrisch leitfähiges Material enthält oder daraus besteht, und das/die eine auf dem elektrisch leitfähigen Material des Grundkörpers aufgebrachte Calciumhydroxid-Schicht aufweist. Das Implantat oder die Implantatkomponente ist dadurch gekennzeichnet, dass die Calciumhydroxid-Schicht Calciumphosphat enthält und zwar in einem Gewichtsprozent-Anteil, der geringer ist als der Gewichtsprozent-Anteil an Calciumhydroxid in dieser Schicht. Es wird ferner ein Verfahren zur Bereitstellung des erfindungsgemäßen Implantats oder der erfindungsgemäßen Implantatkomponente vorgestellt. Das bereitgestellte Implantat bzw. die bereitgestellte Implantatkomponente zeichnen sich dadurch aus, dass sie eine lokale und temporäre antimikrobielle Wirkung aufweisen, eine Ausbildung Antibiotika-resistenter Keime vermeiden, auf Knochenmaterial wachstumsfördernd wirken und keine negativen Nebenwirkungen im Körper hervorrufen.

Implantatinfektionen sind ein schwerwiegendes Problem in der chirurgischen Versorgung, unter anderem bei Hüft- und Knieimplantaten. Laut dem Australischen Nationalen Register für Gelenkimplantate liegt die Infektionsrate nach dem Einsetzen von Implantaten zwischen einem und drei Prozent aller Operationen. Bei Primäroperationen sind Infektionsraten von zweieinhalb Prozent und bei Revisionsoperationen sind sogar Infektionsraten von bis zu zehn Prozent beobachtet worden. Da Patienten mit Gelenkimplantaten immer jünger werden, wird sich zukünftig die Anzahl von Revisionsoperationen und damit das Risiko einer Infektion nach Einsetzen von Implantaten erhöhen.

Bei Implantatinfektionen handelt es sich um schwerwiegende Komplikationen mit erhöhtem Sterblichkeitsrisiko und erheblichen sozio-ökonomischen Kosten. Sie erfordern die Entfernung des Implantats aus dem Körper und seinen Ersatz durch ein neues Implantat, also einen erneuten operativen Eingriff. Im Zusammenhang mit dem operativen Eingriff beim (erneuten) Einsatz eines Implantats kann es zu verschiedenen Krankheitsfolgen kommen. Infektionen sind möglich, da Operationen nicht komplett steril ablaufen und eine Infektion oft durch Berührung an den Wundrändern, hämatogen oder aerogen (siehe Gravius, S., Wirtz, D.C., Orthopädie, 2015, Bd. 44, S. 952-960) erfolgt, welche auch durch die Operationsvorbereitung nicht vollständig frei von Keimen sind. Die Infektion kann oft bereits bei einer lediglich geringen Keimzahl erfolgen und wird in den meisten Fällen vom Immunsystem des Patienten und der antibiotischen Prophylaxe beherrscht. Gerade bei Risikopatienten mit Komorbiditäten ist dies jedoch oft nicht der Fall und das Infektionsrisiko ist bei diesen Patienten deutlich erhöht.

Zur Verminderung des Infektionsrisikos bei Implantaten werden im Stand der Technik verschiedene Wege beschritten.

Es ist bekannt, Implantate mit Antibiotika zu beschichten (Schmidmaier, G. et al., Injury, 2006, Band 37, Suppl. 2, S105-S112) oder Antibiotika in eine Beschichtung auf dem Implantat einzuarbeiten (Hetrick, E.M. et al., Chem. Soc. Rev., 2006, Band 35, Ausgabe 9, S. 780-789), wobei die Antibiotika von der Beschichtung im Lauf der Zeit langsam freigesetzt werden. Bei Implantaten, welche mit Hilfe von Zement im Knochen fixiert werden, kann auch der Zement mit Antibiotika angereichert werden (Parvizi, J. et al, Acta Orthopaedica, 2008, Band 79, S. 335-341). Die Verwendung von Antibiotika ist teilweise problematisch, da am Implantat befindliche Keime bereits resistent gegen die eingesetzten Antibiotika sein können und das Antibiotikum somit unwirksam ist. Zudem bleiben die über einen längeren Zeitraum aus der Schicht freigesetzten Antibiotika für eine lange Zeit im Körper und können so auch selbst zu Resistenzbildungen bei Bakterienstämmen beitragen. Die Resistenzbildung kann auch Bakterienstämme betreffen, die für das Einwachsen des Implantates gar keine Bedeutung haben.

Seit ein paar Jahren wird eine Impregnation der Implantatoberfläche mit Silberpartikeln, teilweise als Silber-Nanopartikel, zur Verhinderung von Infektionen eingesetzt (Knetch, M.L.W. et al., Polymers, 2011, Band 3, S. 340-366). Der Einsatz von Silber ist jedoch problematisch, da bis heute nicht vollständig verstanden ist, welche - vielleicht ungewollten - Wirkungen die Silberpartikel im Körper entfalten und welche Langzeitwirkungen sie dabei haben.

Aus diesen Gründen gibt es eine starke Motivation, eine antibakterielle Ausrüstung von Implantatoberflächen oder Implantatkomponentenoberflächen auf eine Weise zu realisieren, die eine zuverlässige, hohe antimikrobielle Wirksamkeit besitzen, aber keine schädlichen Nebenwirkungen im Körper hervorrufen. Idealerweise sollten die Implantate bzw. die Implantatkomponenten zudem eine wachstumsfördernde Wirkung für Knochenmaterial aufweisen, da somit der Heilungsprozess beschleunigt wird.

Die EP 1 975 277 A1 offenbart ein Oberflächenbehandlungsverfahrens für Titan oder eine Titanlegierung zur schnelleren Abscheidung von Apatit auf einer Oberfläche des Titans bzw. der Titanlegierung, wobei bei dem Verfahren Titan oder eine Titanlegierung mit einer Lösung eines Calciumsalzes in Kontakt gebracht wird, die kein gelöstes oder suspendiertes Calciumhalogenid enthält.

Die DE 101 58 302 A1 offenbart ein Verfahren zur Beschichtung von implantierbaren Metallkörpern mit bioaktiven Materialien, die Calciumphosphat enthalten, wobei der Metallkörper in einer wässrigen Calcium- und PhosphatIonen enthaltenen Behandlungslösung als Kathode geschaltet wird und diese Polarisierung für 0,1 bis 120 min aufrechterhalten wird.

Die US 2002/143398 A1 offenbart ein biokompatibles Implantat zur medizinischen Verwendung und ein Verfahren zu dessen Herstellung, wobei in dem Verfahren das Implantat elektrochemisch behandelt wird, indem es als Anode in einer Calciumionen-enthaltenden Elektrolytlösung verwendet wird, um zunächst eine Schicht aus Oxidhydraten oder Oxidgelen zu erzeugen, und anschließend das Implantat als Kathode in einer anderen elektrochemischen Oberflächenbehandlung verwendet wird, um eine Schicht enthaltend Calciumionen zu erzeugen, sodass auf dem Implantat am Ende des Verfahrens eine hydratisierte Gelschicht zur Bildung von Apatit vorliegt.

Ausgehend hiervon war es die Aufgabe der vorliegenden Erfindung, ein Implantat bzw. eine Implantatkomponente bereitzustellen, das die im Stand der Technik bekannten Nachteile nicht aufweist. Insbesondere sollte das Implantat bzw. die Implantatkomponente eine nur lokale und nur temporäre antimikrobielle Wirkung aufweisen, eine Ausbildung Antibiotika-resistenter Keime vermeiden, eine wachstumsfördernde Wirkung für Knochenmaterial ausüben und dabei keine Nebenwirkungen im Körper hervorrufen.

Die Aufgabe wird gelöst durch das Implantat und/oder die Implantatkomponente mit den Merkmalen von Anspruch 1 und durch das Verfahren zur Herstellung eines Implantats und/oder einer Implantatkomponente mit den Merkmalen von Anspruch 9. Die abhängigen Ansprüche zeigen vorteilhafte Weiterbildungen auf.

Erfindungsgemäß wird ein Implantat oder eine Implantatkomponente bereitgestellt, enthaltend
a) einen Grundkörper eines Implantats oder einer Implantatkomponente, der zumindest an einer Oberfläche ein elektrisch leitfähiges Material enthält oder daraus besteht; und
b) eine Calciumhydroxid-Schicht, die Calciumhydroxid (Ca(OH)₂) enthält oder daraus besteht, wobei die Calciumhydroxid-Schicht auf dem elektrisch leitfähigen Material des Grundkörpers aufgebracht ist;
dadurch gekennzeichnet, dass die Calciumhydroxid-Schicht Calciumphosphat in einem Gewichtsprozent-Anteil enthält, der geringer ist als der Gewichtsprozent-Anteil an Calciumhydroxid, wobei sich der Gewichtsprozent-Anteil auf das Gesamtgewicht der Calciumhydroxid-Schicht bezieht.

Die Calciumhydroxid-Schicht des Implantats oder der Implantatkomponente bewirkt, dass lediglich lokal und lediglich zeitlich begrenzt durch eine pH-Wert-Erhöhung eine antimikrobielle (z.B. bakterizide) Wirkung ohne Nebenwirkungen im Körper erzielt wird, eine Ausbildung Antibiotika-resistenter Keime vermieden wird und eine wachstumsfördernde Wirkung für Knochenmaterial bereitgestellt wird (positive osseointegrative Eigenschaften). Grund der lediglich temporären und lokalen Wirkung ist, dass die Wirkstoffe der Calciumhydroxid-Schicht vom Körper an Ort und Stelle nach einer gewissen Zeit durch Körperflüssigkeiten vollständig aufgelöst und umgewandelt werden.

Die Wirkung der Calciumhydroxid-Schicht ist dabei jedoch ausreichend lang und hoch, um nach einem Einsetzen des Implantats oder der Implantatkomponente in den Körper für eine ausreichend lange Zeit eine antimikrobielle Wirkung sicherzustellen und etwaige Keime abzutöten, die beispielsweise während des Einsetzens des Implantats oder der Implantatkomponente (bei einer Operation) in den Körper gelangen. Durch die lediglich temporäre Wirkung der Calciumhydroxid-Schicht werden Langzeitrisiken für die Gesundheit des Patienten vermieden. Selbst wenn nicht alle Keime durch die Calciumhydroxid-Schicht abgetötet werden sollten, wird die Keimzahl durch die Wirkung der Calciumhydroxid-Schicht so stark reduziert, dass das körpereigene Immunsystem die Bekämpfung der verbliebenen Keime problemlos übernehmen kann.

Das neben dem Calciumhydroxid in der Calciumhydroxid-Schicht vorhandene Calciumphosphat wirkt ebenfalls bakterizid, unterstützt die Wirkung der pH-Wert-Erhöhung durch das Calciumhydroxid und hat eine wachstumsfördernde Wirkung für Knochenmaterial in der Umgebung des Implantats oder der Implantatkomponente. Das Calciumphosphat unterstützt damit die Osseointegration des Implantats oder der Implantatkomponente und bewirkt somit nach dessen Platzierung im Körper eine schnellere und zuverlässigere Integration im Körper.

Das erfindungsgemäße Implantat oder die erfindungsgemäße Implantatkomponente erzielen die antimikrobielle Wirkung ohne die Verwendung von Antibiotika oder von antibakteriell wirkenden Metallen, wie beispielsweise Silber, Kupfer oder Strontium. Es wird somit verhindert, dass es nach dessen Platzierung im Körper zur Ausbildung von Antibiotika-resistenten Keimen kommen kann. Zudem kann die antibakteriell wirkende Calciumhydroxid-Schicht vom Körper vollständig resorbiert werden, sodass - anders als bei Antibiotika-Beschichtungen oder Metall-Beschichtungen - keine Langzeitrisiken für die Gesundheit des Patienten zu befürchten sind.

Das in der Calciumhydroxid-Schicht enthaltene Calciumphosphat kann ein Calciumphosphat, das ausgewählt ist aus der Gruppe bestehend aus Calciumdihydrogenphosphat ("Monocalciumphosphat" = Ca(H₂PO₄)₂), Calciumhydrogenphosphat ("Dicalciumphosphat" = CaHPO₄), Calciumphosphat ("Tricalciumphosphat" = Ca₃(PO₄)₂), Hydroxyapatit (Ca₅[OH|(PO₄)₃]), Brushit (CaH-PO₄·2H₂O) und Kombinationen hiervon, enthalten oder daraus bestehen. Bevorzugt enthält das Calciumphosphat der Calciumhydroxid-Schicht Hydroxyapatit und/oder Brushit, oder besteht daraus.

Das Implantat oder die Implantatkomponente kann dadurch gekennzeichnet sein, dass der Grundkörper ein Material enthält oder daraus besteht, das ausgewählt ist aus der Gruppe bestehend aus Metallen, Halbmetallen, Kohlenstoffen, Kunststoffen und Keramiken. Bevorzugt ist das Material ausgewählt aus der Gruppe der Metalle. Insbesondere handelt es sich bei dem Material um Titan, eine Titanlegierung oder Cobalt-Chrom-Stahl.

Das elektrisch leitfähige Material des Grundkörpers kann auf dem Grundkörper aufgebracht sein oder monolithisch (einstückig) mit dem Grundkörper sein. Es kann ein zum Grundkörper verschiedenes Material enthalten oder daraus bestehen oder ein zum Grundkörper identisches Material enthalten oder daraus bestehen.

Unter der "Oberfläche" des Grundkörpers, die das elektrisch leitfähige Material enthält oder daraus besteht, wird somit erfindungsgemäß eine Oberfläche des Grundkörpers verstanden, auf die entweder das elektrisch leitfähige Material (über ein bestimmtes Verfahren) aufgebracht wird (Zweistückigkeit zwischen Grundkörper und elektrisch leitfähigen Material) oder bei der das elektrisch leitfähige Material (bereits) ein integrativer Bestandteil ist (Einstückigkeit zwischen Grundkörper und elektrisch leitfähigen Material).

Als elektrisch leitfähiges Material werden im Sinne dieser Erfindung auch Materialien verstanden, die von ihrer chemischen Beschaffenheit elektrisch leitfähig sind und bei Sauerstoffkontakt (z.B. über Luftkontakt oder Wasserkontakt) an ihrer Oberfläche eine oxidative Schutzschicht ausbilden, die nicht elektrisch leitfähig ist. Hierunter fallen beispielsweise die Materialien Titan und/oder Titanlegierungen.

Das elektrisch leitfähige Material des Grundkörpers kann ausgewählt sein aus der Gruppe bestehend aus Metallen, Halbmetallen, Kohlenstoffen und Kunststoffen. Bevorzugt ist das elektrisch leitfähige Material ausgewählt aus der Gruppe der Metalle, besonders bevorzugt ausgewählt aus der Gruppe bestehend aus Titan, Titanlegierung, Tantal, Magnesium und Legierungen hiervon. Insbesondere handelt es sich bei dem elektrisch leitfähigen Material um Titan oder eine Titanlegierung.

Auf dem elektrisch leitfähigen Material des Grundkörpers kann eine Calciumphosphat-Schicht aufgebracht sein, die Calciumphosphat enthält oder daraus besteht.

Das Calciumphosphat der Calciumphosphat-Schicht kann ein Calciumphosphat, das ausgewählt ist aus der Gruppe bestehend aus Calciumdihydrogenphosphat ("Monocalciumphosphat" = Ca(H₂PO₄)₂), Calciumhydrogenphosphat ("Dicalciumphosphat" = CaHPO₄), Calciumphosphat ("Tricalciumphosphat" = Ca₃(PO₄)₂), Hydroxyapatit (Ca₅[OH|(PO₄)₃]), Brushit (CaHPO₄·2H₂O) und Kombinationen hiervon enthalten oder daraus bestehen. Bevorzugt enthält das Calciumphosphat der Caciumphosphat-Schicht Hydroxyapatit und/oder Brushit, oder besteht daraus. Eine solche Calciumphosphat-Schicht kann beispielsweise wie in Dorozhkin, S.V. et al. (Progress in biomaterials, Band 1, Ausgabe 1, S. 1ff.) beschrieben bereitgestellt werden. Die Calciumphosphat-Schicht hat den Vorteil, dass die Verbesserung des Einwachsverhaltens (d.h. die positiven osseointegrativen Eigenschaften), die bereits durch den Anteil an Calciumphosphat in der Calciumhydroxid-Schicht bereitgestellt wird, noch weiter verbessert wird.

Die Calciumphosphat-Schicht enthält Calciumphosphat bevorzugt in einem Gewichtsprozent-Anteil, der höher ist als ein Gewichtsprozentanteil an Calciumhydroxid in dieser Schicht, wobei sich der Gewichtsprozent-Anteil auf das Gesamtgewicht der Calciumphosphat-Schicht bezieht.

In einer bevorzugten Ausgestaltungsform enthält die Calciumphosphat-Schicht >50 Gew.-% Calciumphosphat, bezogen auf das Gesamtgewicht der Calciumphosphat-Schicht.

In einer bevorzugten Ausgestaltungsform kontaktiert die Calciumphosphat-Schicht das elektrisch leitfähige Material des Grundkörpers. Die Calciumphosphat-Schicht kann hierbei das elektrisch leitfähige Material des Grundkörpers (z.B. eine Titan-Spritzschicht) überziehen. Dabei wird die Rauheit des elektrisch leitfähigen Materials ein wenig verringert. Die Morphologie der elektrisch leitfähigen Schicht verändert sich hierbei jedoch nur geringfügig. Zudem ist die Oberfläche in ihrer geometrischen Struktur noch immer selbstähnlich, d.h. stark gegliedert, vielfältig gebrochen und auf verschiedenen Größenskalen wiederkehrende Geometrieelemente enthaltend.

In einer weiteren bevorzugten Ausgestaltungsform kontaktiert die Calciumphosphat-Schicht die Calciumhydroxid-Schicht.

Die Calciumphosphat-Schicht kann über eine elektrochemische Abscheidung oder einen Plasmaspritzprozess auf dem elektrisch leitfähigen Material des Grundkörpers abgeschieden sein.

Die Calciumphosphat-Schicht liegt bevorzugt als Schicht vor, die eine gleiche Schichtdicke über die gesamte Ausdehnung der Schicht aufweist.

Zudem kann die Calciumphosphat-Schicht als poröse Schicht vorliegen.

In einer bevorzugten Ausgestaltungsform weist die Calciumphosphat-Schicht eine Schichtdicke im Bereich von 2 bis 500 µm, bevorzugt im Bereich von 5 bis 200 µm, besonders bevorzugt im Bereich von 10 bis 130 µm auf.

Die Calciumhydroxid-Schicht des Implantats oder der Implantatkomponente kann über eine elektrochemische Abscheidung abgeschieden sein, bevorzugt über eine elektrochemische Abscheidung mit einem Elektrolyten, der Calciumnitrat, Ammoniumdihydrogenphosphat und Citronensäure enthält und besonders bevorzugt einen pH Wert von < 7 aufweist. Die elektrochemisch abgeschiedene Calciumhydroxid-Schicht hat - verglichen mit anderen Abscheideverfahren - den Vorteil, dass die Schicht auch in etwaige Poren und Kavitäten des elektrisch leitfähigen Materials des Grundkörpers abgeschieden wird und somit eine gute Haftfestigkeit aufweist. Ferner weist eine elektrochemisch abgeschiedene Schicht eine hohe Homogenität, eine feine Schichtstruktur und eine konstante Schichtdicke über die gesamte Ausdehnung der Schicht auf. Folglich kann durch die elektrochemische Abscheidung auf zuverlässige Art und Weise eine homogene Wirksamkeit gewährleistet werden. Darüber hinaus kann der oberste Bereich der über elektrochemische Abscheidung aufgebrachten Schicht nach Einbringen des Implantats oder der Implantatkomponente in den Körper sehr leicht von Körperflüssigkeiten gelöst werden. Folglich kann der Effekt der pH-Wert-Erhöhung, und damit der bakteriziden Wirkung, sehr schnell nach dem Einbringen in den Körper einsetzen.

Die Calciumhydroxid-Schicht liegt bevorzugt als Schicht vor, die eine gleiche Schichtdicke über die gesamte Ausdehnung der Schicht aufweist.

In einer bevorzugten Ausgestaltungsform weist die Calciumhydroxid-Schicht eine Schichtdicke im Bereich von 1 bis 50 µm, bevorzugt im Bereich von 2 bis 40 µm, besonders bevorzugt im Bereich von >20 bis 30 µm (z.B. im Bereich von 22 bis 30 µm), auf. Je höher die Schichtdicke der Calciumhydroxid-Schicht ist, desto stärker und länger anhaltend ist die antimikrobielle Wirkung dieser Schicht. Grund hierfür ist, dass eine dickere Schicht in einem gegebenen Flüssigkeitsvolumen im Körper einen höheren pH-Wert erzeugen kann und länger stabil bleibt und damit an Ort und Stelle eine stärkere und länger anhaltende antimikrobielle Wirkung entfalten kann.

In einer bevorzugten Ausgestaltungsform liegt der Anteil von Calciumhydroxid in der Calciumhydroxid-Schicht im Bereich von >50 Gew.-% und <100 Gew.-%, bezogen auf das Gesamtgewicht der Calciumhydroxid-Schicht. Folglich liegt der Anteil an Calciumphosphat bevorzugt im Bereich von >0 Gew.-% bis ≤50 Gew.-%, bezogen auf das Gesamtgewicht der Calciumhydroxid-Schicht. In der Calciumhydroxid-Schicht liegt der Anteil an Calciumphosphat besonders bevorzugt im Bereich von >0 Gew.-% und <40 Gew.-%, bevorzugt von >2 Gew.-% und <20 Gew.-%, bezogen auf das Gesamtgewicht der Calciumhydroxid-Schicht.

Besonders bevorzugt liegt das Calciumphosphat in der Calciumhydroxid-Schicht in Form von Partikeln vor, insbesondere in Form von Partikeln mit einer Partikelgröße von <1 µm. Die Partikelgröße kann beispielsweise über Elektronenmikroskopie bestimmt werden.

In einer bevorzugten Ausgestaltungsform ist das erfindungsgemäße Implantat oder die erfindungsgemäße Implantatkomponente zur Verwendung in der Medizin, bevorzugt zur Verwendung in der Chirurgie, vorgesehen.

Erfindungsgemäß wird ferner ein Verfahren zur Herstellung eines Implantats oder einer Implantatkomponente vorgestellt. Das Verfahren umfasst die Schritte
a) Bereitstellen eines Grundkörpers eines Implantats oder einer Implantatkomponente, wobei der Grundkörper zumindest an einer Oberfläche ein elektrisch leitfähiges Material enthält oder daraus besteht; und
b) Aufbringen einer Calciumhydroxid-Schicht, die Calciumhydroxid (Ca(OH)₂) enthält oder daraus besteht, auf dem elektrisch leitfähigen Material des Grundkörpers;
dadurch gekennzeichnet, dass die Calciumhydroxid-Schicht so aufgebracht wird, dass sie Calciumphosphat in einem Gewichtsprozent-Anteil enthält, der geringer ist als der Gewichtsprozent-Anteil an Calciumhydroxid, wobei sich der Gewichtsprozent-Anteil auf das Gesamtgewicht der Calciumhydroxid-Schicht bezieht.

Mit dem Verfahren wird eine Calciumhydroxid-Schicht erzeugt, welche zum einen Teil Calciumhydroxid und zum anderen Teil Calciumphosphat enthält oder daraus besteht. Das in der Schicht enthaltene Calciumphosphat unterstützt gleichzeitig die Osseointegration.

Das in der Calciumhydroxid-Schicht enthaltene Calciumphosphat kann Calciumphosphat, das ausgewählt ist aus der Gruppe bestehend aus Calciumdihydrogenphosphat ("Monocalciumphosphat" = Ca(H₂PO₄)₂), Calciumhydrogenphosphat ("Dicalciumphosphat" = CaHPO₄), Calciumphosphat ("Tricalciumphosphat" = Ca₃(PO₄)₂), Hydroxyapatit (Ca₅[OH|(PO₄)₃]), Brushit (CaHPO₄·2H₂O) und Kombinationen hiervon, enthalten oder daraus bestehen. Bevorzugt enthält das Calciumphosphat der Calciumhydroxid-Schicht Hydroxyapatit und/oder Brushit, oder besteht daraus.

In dem Verfahren kann ein Grundkörper bereitgestellt werden, der ein Material enthält oder daraus besteht, das ausgewählt ist aus der Gruppe bestehend aus Metallen, Halbmetallen, Kohlenstoffen, Kunststoffen und Keramiken. Das Material ist bevorzugt ausgewählt aus der Gruppe der Metalle, besonders bevorzugt ausgewählt aus der Gruppe der Leichtmetalle. Insbesondere handelt es sich bei dem Material um Titan oder eine Titanlegierung.

Ferner kann in dem Verfahren ein Grundkörper bereitgestellt werden, dessen elektrisch leitfähiges Material auf dem Grundkörper aufgebracht ist oder monolithisch (einstückig) mit dem Grundkörper ist. Das elektrisch leitfähige Material kann ein zum Grundkörper verschiedenes Material enthalten oder daraus bestehen oder ein zum Grundkörper identisches Material enthalten oder daraus bestehen.

Das elektrisch leitfähige Material kann ausgewählt sein aus der Gruppe bestehend aus Metallen, Halbmetallen, Kohlenstoffen und Kunststoffen. Bevorzugt ist das elektrisch leitfähige Material ausgewählt aus der Gruppe der Metalle, besonders bevorzugt aus der Gruppe bestehend aus Titan, Titanlegierung, Tantal, Magnesium und Legierungen hiervon. Insbesondere handelt es sich bei dem elektrisch leitfähigen Material um Titan oder eine Titanlegierung.

In einer bevorzugten Ausführungsform des Verfahrens wird auf das elektrisch leitfähige Material des Grundkörpers eine Calciumphosphat-Schicht aufgebracht, die Calciumphosphat enthält oder daraus besteht.

Das Calciumphosphat der Calciumphosphat-Schicht kann Calciumphosphat, das ausgewählt ist aus der Gruppe bestehend aus Calciumdihydrogenphosphat ("Monocalciumphosphat" = Ca(H₂PO₄)₂), Calciumhydrogenphosphat ("Dicalciumphosphat" = CaHPO₄), Calciumphosphat ("Tricalciumphosphat" = Ca₃(PO₄)₂), Hydroxyapatit (Ca₅[OH|(PO₄)₃]), Brushit (CaHPO₄·2H₂O) und Kombinationen hiervon, enthalten oder daraus bestehen. Bevorzugt enthält das Calciumphosphat der Calciumphosphat-Schicht Hydroxyapatit und/oder Brushit, oder besteht daraus. Eine solche Calciumphosphat-Schicht kann beispielsweise über ein Verfahren erzeugt werden, wie es in Dorozhkin, S.V. et al. (Progress in biomaterials, Band 1, Ausgabe 1, S. 1ff.) beschrieben ist. Die Calciumphosphat-Schicht hat den Vorteil, dass die Verbesserung des Einwachsverhaltens (d.h. die positiven osseointegrativen Eigenschaften), die bereits durch den Anteil an Calciumphosphat in der Calciumhydroxid-Schicht bereitgestellt wird, noch weiter verbessert wird.

Die Calciumphosphat-Schicht enthält Calciumphosphat bevorzugt in einem Gewichtsprozent-Anteil, der höher ist als ein Gewichtsprozentanteil an Calciumhydroxid in dieser Schicht, wobei sich der Gewichtsprozent-Anteil auf das Gesamtgewicht der Calciumphosphat-Schicht bezieht.

In einer bevorzugten Ausführungsform enthält die Calciumphosphat-Schicht >50 Gew.-% Calciumphosphat, bezogen auf das Gesamtgewicht der Calciumphosphat-Schicht.

In einer bevorzugten Ausführungsform wird die Calciumphosphat-Schicht so auf dem Grundkörper aufgebracht, dass sie das elektrisch leitfähige Material des Grundkörpers kontaktiert. Hierbei kann die Calciumphosphat-Schicht das elektrisch leitfähige Material des Grundkörpers (z.B. eine Titan-Spritzschicht) überziehen. Die Rauheit des elektrisch leitfähigen Materials verringert sich dadurch ein wenig. Die Morphologie der elektrisch leitfähigen Schicht wird jedoch nur geringfügig verändert. Zudem ist die Oberfläche in ihrer geometrischen Struktur noch immer selbstähnlich, d.h. stark gegliedert, vielfältig gebrochen und auf verschiedenen Größenskalen wiederkehrende Geometrieelemente enthaltend.

In einer weiteren bevorzugten Ausführungsform wird die Calciumphosphat-Schicht so auf dem Grundkörper aufgebracht, dass sie die Calciumhydroxid-Schicht kontaktiert.

Die Calciumphosphat-Schicht kann über eine elektrochemische Abscheidung oder einen Plasmaspritzprozess auf dem elektrisch leitfähigen Material des Grundkörpers abgeschieden werden.

Die Calciumphosphat-Schicht wird bevorzugt als Schicht aufgebracht, die eine gleiche Schichtdicke über die gesamte Ausdehnung der Schicht aufweist.

Zudem kann die Calciumphosphat-Schicht als poröse Schicht aufgebracht werden.

In einer bevorzugten Ausführungsform wird die Calciumphosphat-Schicht bis zu einer Schichtdicke im Bereich von 2 bis 500 µm, bevorzugt im Bereich von 5 bis 200 µm, besonders bevorzugt im Bereich von 10 bis 130 µm, aufgebracht.

Die in dem Verfahren aufgebrachte Calciumhydroxid-Schicht kann über eine elektrochemische Abscheidung abgeschieden sein, bevorzugt über eine elektrochemische Abscheidung mit einem Elektrolyten, der Calciumnitrat, Ammoniumdihydrogenphosphat und Citronensäure enthält und besonders bevorzugt einen pH Wert von < 7 aufweist. Dieses Verfahren hat den Vorteil, dass die Schicht auch in etwaige Poren und Kavitäten des elektrisch leitfähigen Materials des Grundkörpers abgeschieden wird und somit eine gute Haftfestigkeit aufweist. Ferner weist die elektrochemisch abgeschiedene Schicht eine hohe Homogenität, eine feine Schichtstruktur und eine konstante Schichtdicke über die gesamte Ausdehnung der Schicht auf. Folglich kann durch die elektrochemische Abscheidung auf zuverlässige Art und Weise eine homogene Wirksamkeit gewährleistet werden. Darüber hinaus kann der oberste Bereich der über elektrochemische Abscheidung aufgebrachten Schicht nach Einbringen des Implantats oder der Implantatkomponente in den Körper sehr leicht von Körperflüssigkeiten gelöst werden. Somit kann der Effekt der pH-Wert-Erhöhung, und damit der bakteriziden Wirkung, sehr schnell nach dem Einbringen in den Körper einsetzen.

Die Calciumhydroxid-Schicht wird in dem Verfahren bevorzugt als Schicht aufgebracht, die eine gleiche Schichtdicke über die gesamte Ausdehnung der Schicht aufweist.

In einer bevorzugten Ausführungsform wird die Calciumhydroxid-Schicht bis zu einer Schichtdicke im Bereich von 1 bis 50 µm, bevorzugt im Bereich von 2 bis 40 µm, besonders bevorzugt im Bereich von >20 bis 30 µm (z.B. 22 bis 30 µm), aufgebracht.

In einer bevorzugten Ausgestaltungsform liegt der Anteil von Calciumhydroxid in der Calciumhydroxid-Schicht im Bereich von >50 Gew.-% und <100 Gew.-%, bezogen auf das Gesamtgewicht der Calciumhydroxid-Schicht. Folglich liegt der Anteil an Calciumphosphat bevorzugt im Bereich von >0 Gew.-% bis ≤50 Gew.-%, bezogen auf das Gesamtgewicht der Calciumhydroxid-Schicht. Die Calciumhydroxid-Schicht wird besonders bevorzugt so aufgebracht, dass der Anteil an Calciumphosphat in der Calciumhydroxid-Schicht im Bereich von >0 Gew.-% und <40 Gew.-%, bevorzugt von >2 Gew.-% und <20 Gew.-%, bezogen auf das Gesamtgewicht der Calciumhydroxid-Schicht, liegt.

Besonders bevorzugt wird die Calciumhydroxid-Schicht so aufgebracht, dass das Calciumphosphat in Form von Partikeln in der Calciumhydroxid-Schicht vorliegt.

In einer bevorzugten Ausführungsform wird die Calciumhydroxid-Schicht so aufgebracht, dass das Calciumphosphat in Form von Partikeln mit einer Partikelgröße im Bereich von < 1 µm vorliegt.

Es ist besonders bevorzugt, dass mit dem hier vorgestellten Verfahren ein erfindungsgemäßes Implantat oder eine erfindungsgemäße Implantatkomponente hergestellt wird.

Anhand der nachfolgenden Figur und der nachfolgenden Beispiele soll der erfindungsgemäße Gegenstand näher erläutert werden, ohne diesen auf die hier gezeigten, spezifischen Ausführungsformen einschränken zu wollen.

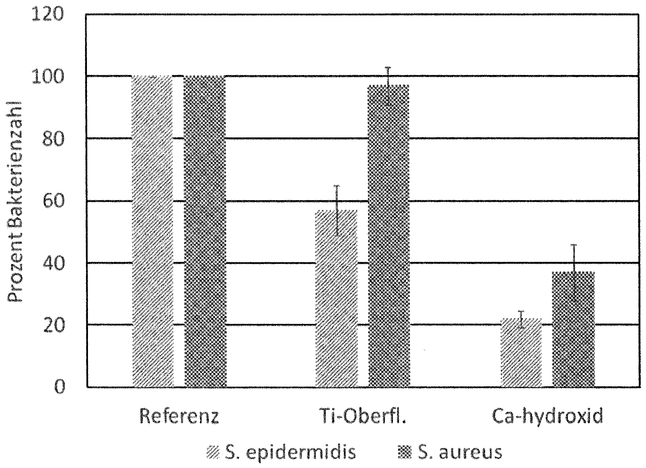

Die Figur zeigt das Ergebnis eines Experiments, bei dem der Grad der Reduktion der Bakterienanzahl von zwei Bakterienstämmen (S. *epidermidis* und S. *aureus*) in Abhängigkeit von der Oberfläche eines Implantats, auf das die Bakterienstämme jeweils appliziert wurden, gemessen wurde. Bei der Referenz handel es sich um ein Implantat mit einer Polyethylen-Oberfläche (ohne bakterienhemmende Eigenschaften). Es wurde gefunden, dass eine Implantatoberfläche aus Titan nur bei *S. epidermidis* zu einer gewissen Reduktion der Bakterienzahl führt. Eine Oberfläche, die Calciumhydroxid enthielt, führte jedoch bei beiden Bakterienstämmen zu einer deutlichen Reduktion der Bakterienzahl.

### Beispiel 1 - Herstellung eines erfindungsgemäßen Implantats ohne Calciumphosphat-Zwischenschicht

Es wird ein Implantat verwendet, dessen Grundkörper aus einer Titanlegierung besteht. Der Grundkörper wird im Vakuum mit zwei Schichten aus Reintitan beschichtet. Die erste Schicht (Haftschicht) hat eine Stärke von 30 µm und die zweite Schicht hat eine Stärke von 150 µm. Diese Titanspritzschicht bildet eine elektrisch leitfähige Schicht und wird anschließend in einem wässrigen Elektrolyt mit einer Calciumhydroxid-Schicht versehen.

Die Zusammensetzung des wässrigen Elektrolyten sowie die verwendeten Prozessparameter sind in Tabelle 1 aufgelistet.

**Tabelle 1**

| | | |
|---|---|---|
| Elektrolyt | Ca(NO₃)₂ | 75 mmol/l |
| | (NH₄)H₂PO₄ | 40 mmol/l |
| | Citronensäure | 20 mmol/l |
| Prozesstemperatur | | 60 °C |
| Stromdichte | | 90 mA/cm² |
| Zeit | | 20 min. |
| Schichtdicke | | 15 µm |

Die abgeschiedene Calciumhydroxid-Schicht enthält neben Calciumhydroxid auch Calciumphosphat. Grund hierfür ist, dass Calciumhydroxid eine Zwischenphase bei der elektrochemischen Abscheidung von Calciumphosphat darstellt. Die Anwesenheit der Citronensäure in dem Elektrolyt unterdrückt zwar die Bildung von Calciumphosphat, aber in der angegebenen Konzentration nur so wenig, dass eine Calciumhydoxid-Schicht entsteht, die immer noch Calciumphosphat enthält. Der Anteil an Calciumphosphat ist jedoch geringer als der Anteil an Calciumhydroxid.

Das Verhältnis der beiden Materialkomponenten lässt sich durch Änderung der eingesetzten Citronensäuremenge verschieben. Das bedeutet, dass bei einer höherer Konzentration von Citronensäure (bzw. Citrationen) in dem Elektrolyten die Menge an abgeschiedenem Calciumphosphat abnimmt und bei einer geringeren Konzentration von Citronensäure (bzw. Citrationen) die Menge an abgeschiedenem Calciumphosphat zunimmt. Durch einen höheren Anteil an Calciumphosphat wird die bakterizide Wirkung ein wenig geringer, die osseointegrative Wirkung des Calciumphosphats nimmt jedoch zu, was erfindungsgemäß gewünscht ist. Sehr hohe Konzentrationen an Citrationen im Elektrolyt sind auch deshalb nachteilig, da diese einen geringeren pH-Wert im wässrigen Elektrolyt erzeugen und somit eine schnellere Rücklösung der abgeschiedenen Schicht bewirken, wodurch die maximal erreichbare Schichtdicke abnimmt, bevor sich ein dynamisches Gleichgewicht einstellt.

Der in Tabelle 1 beschriebene Elektrolyt weist eine Konzentration von CitratIonen auf, die vorteilhaft ist, da sie einerseits einen hohen Gehalt an Calciumhydroxid in der Schicht liefert und andererseits sicherstellt, dass nur eine geringe Rücklösung der abgeschiedenen Schicht bewirkt wird und die abgeschiedene Schicht auch Calciumphosphat enthält.

### Beispiel 2 - Herstellung eines erfindungsgemäßen Implantats mit Calciumphosphat-Zwischenschicht

Es wird ein Implantat verwendet, dessen Grundkörper aus einer Titanlegierung besteht. Der Grundkörper wird im Vakuum mit zwei Schichten aus Reintitan beschichtet. Die erste hat eine Stärke von 30 µm und die zweite Schicht hat eine Stärke von 50 µm. Diese Titanspritzschicht bildet eine elektrisch leitfähige Schicht und wird anschließend im Vakuum mit einer Calciumphosphat-Schicht mit einer Stärke von 100 µm versehen.

Das mit der Calciumphosphat-Schicht ausgestattete Implantat wird anschließend in einem wässrigen Elektrolyt mit einer Calciumhydroxid-Schicht versehen.

Die Zusammensetzung des wässrigen Elektrolyten sowie die verwendeten Prozessparameter sind in Tabelle 2 aufgelistet.

**Tabelle 2**

| | | |
|---|---|---|
| Elektrolyt | Ca(NO₃)₂ | 75 mmol/l |
| | (NH₄)H₂PO₄ | 40 mmol/l |
| | Citronensäure | 20 mmol/l |
| Prozesstemperatur | | 55 °C |
| Stromdichte | | 110 mA/cm² |
| Zeit | | 20 min. |
| Schichtdicke | | 20 µm |

Die Calciumhydroxid-Schicht weist vergleichbare Eigenschaften zur Calciumhydroxid-Schicht aus Beispiel 1 auf, ist jedoch nicht direkt auf dem elektrisch leitfähigen Material des Grundkörpers abgeschieden, sondern auf der Calciumphosphat-Schicht, die wiederum direkt auf der elektrisch leitfähigen Schicht des Grundkörpers abgeschieden ist.

## Patentansprüche

1. Implantat oder Implantatkomponente, enthaltend
a) einen Grundkörper eines Implantats oder einer Implantatkomponente, der zumindest an einer Oberfläche ein elektrisch leitfähiges Material enthält oder daraus besteht; und
b) eine Calciumhydroxid-Schicht, die Calciumhydroxid enthält oder daraus besteht, wobei die Calciumhydroxid-Schicht auf dem elektrisch leitfähigen Material des Grundkörpers aufgebracht ist;
**dadurch gekennzeichnet, dass** die Calciumhydroxid-Schicht Calciumphosphat in einem Gewichtsprozent-Anteil enthält, der geringer ist als der Gewichtsprozent-Anteil an Calciumhydroxid, wobei sich der Gewichtsprozent-Anteil auf das Gesamtgewicht der Calciumhydroxid-Schicht bezieht.

2. Implantat oder Implantatkomponente gemäß dem vorhergehenden Anspruch, **dadurch gekennzeichnet, dass** das in der Calciumhydroxid-Schicht enthaltene Calciumphosphat ein Calciumphosphat, das ausgewählt ist aus der Gruppe bestehend aus Calciumdihydrogenphosphat, Calciumhydrogenphosphat, Calciumphosphat, Hydroxyapatit, Brushit und Kombinationen hiervon, enthält oder daraus besteht, wobei das in der Calciumhydroxid-Schicht enthaltene Calciumphosphat bevorzugt Hydroxyapatit und/oder Brushit enthält oder daraus besteht.

3. Implantat oder Implantatkomponente gemäß einem der vorhergehenden Ansprüche, **dadurch gekennzeichnet, dass** der Grundkörper ein Material, das ausgewählt ist aus der Gruppe bestehend aus Metallen, Halbmetallen, Kohlenstoffen, Kunststoffen und Keramiken, bevorzugt ausgewählt ist aus der Gruppe der Metalle, besonders bevorzugt ausgewählt ist aus der Gruppe der Leichtmetalle, insbesondere Titan oder eine Titanlegierung, enthält oder daraus besteht.

4. Implantat oder Implantatkomponente gemäß einem der vorhergehenden Ansprüche, **dadurch gekennzeichnet, dass** das elektrisch leitfähige Material des Grundkörpers
i) auf den Grundkörper aufgebracht ist oder einstückig mit dem Grundkörper ist; und/oder
ii) ausgewählt ist aus der Gruppe bestehend aus Metallen, Halbmetallen, Kohlenstoffen und Kunststoffen, bevorzugt ausgewählt ist aus der Gruppe der Metalle, besonders bevorzugt ausgewählt ist aus der Gruppe bestehend aus Titan, Titanlegierung, Tantal, Magnesium und Legierungen hiervon.

5. Implantat oder Implantatkomponente gemäß einem der vorhergehenden Ansprüche, **dadurch gekennzeichnet, dass** auf dem elektrisch leitfähigen Material des Grundkörpers eine Calciumphosphat-Schicht aufgebracht ist, die Calciumphosphat enthält oder daraus besteht, wobei die Calciumphosphat-Schicht bevorzugt Calciumphosphat in einem Gewichtsprozent-Anteil enthält, der höher ist als ein Gewichtsprozentanteil an Calciumhydroxid in dieser Schicht, wobei sich der Gewichtsprozent-Anteil auf das Gesamtgewicht der Calciumphosphat-Schicht bezieht, wobei die Calciumphosphat-Schicht bevorzugt das elektrisch leitfähige Material des Grundkörpers kontaktiert und/oder die Calciumhydroxid-Schicht kontaktiert, wobei die Calciumphosphat-Schicht insbesondere
i) über eine elektrochemische Abscheidung oder einen Plasmaspritzprozess auf dem elektrisch leitfähigen Material des Grundkörpers abgeschieden ist; und/oder
ii) als Schicht vorliegt, die eine gleiche Schichtdicke über die gesamte Ausdehnung der Schicht aufweist; und/oder
iii) als poröse Schicht vorliegt; und/oder
iv) eine Schichtdicke im Bereich von 2 bis 500 µm, bevorzugt im Bereich von 5 bis 200 µm, besonders bevorzugt im Bereich von 10 bis 130 µm, aufweist.

6. Implantat oder Implantatkomponente gemäß einem der vorhergehenden Ansprüche, **dadurch gekennzeichnet, dass** die Calciumhydroxid-Schicht
i) über eine elektrochemische Abscheidung abgeschieden ist, bevorzugt über eine elektrochemische Abscheidung mit einem Elektrolyten, der Calciumnitrat, Ammoniumdihydrogenphosphat und Citronensäure enthält und besonders bevorzugt einen pH Wert von < 7 aufweist; und/oder
ii) als Schicht vorliegt, die eine gleiche Schichtdicke über die gesamte Ausdehnung der Schicht aufweist; und/oder
iii) eine Schichtdicke im Bereich von 1 bis 50 µm, bevorzugt 2 bis 40 µm, besonders bevorzugt >20 bis 30 µm, aufweist.

7. Implantat oder Implantatkomponente gemäß einem der vorhergehenden Ansprüche, **dadurch gekennzeichnet, dass** der Anteil an Calciumphosphat in der Calciumhydroxid-Schicht im Bereich von >0 Gew.-% und <40 Gew.-%, bevorzugt von >2 Gew.-% und <20 Gew.-%, bezogen auf das Gesamtgewicht der Calciumhydroxid-Schicht, liegt, wobei das Calciumphosphat besonders bevorzugt in Form von Partikeln in der Calciumhydroxid-Schicht vorliegt, insbesondere in Form von Partikeln mit einer Partikelgröße von <1 µm.

8. Implantat oder Implantatkomponente gemäß einem der vorhergehenden Ansprüche zur Verwendung in der Medizin, bevorzugt zur Verwendung in der Chirurgie.

9. Verfahren zur Herstellung eines Implantats oder einer Implantatkomponente, umfassend die Schritte
a) Bereitstellen eines Grundkörpers eines Implantats oder einer Implantatkomponente, wobei der Grundkörper zumindest an einer Oberfläche ein elektrisch leitfähiges Material enthält oder daraus besteht; und
b) Aufbringen einer Calciumhydroxid-Schicht, die Calciumhydroxid enthält oder daraus besteht, auf dem elektrisch leitfähigen Material des Grundkörpers;
**dadurch gekennzeichnet, dass** die Calciumhydroxid-Schicht so aufgebracht wird, dass sie Calciumphosphat in einem Gewichtsprozent-Anteil enthält, der geringer ist als der Gewichtsprozent-Anteil an Calciumhydroxid, wobei sich der Gewichtsprozent-Anteil auf das Gesamtgewicht der Calciumhydroxid-Schicht bezieht.

10. Verfahren gemäß Anspruch 9, **dadurch gekennzeichnet, dass** das in der Calciumhydroxid-Schicht enthaltene Calciumphosphat ein Calciumphosphat, das ausgewählt ist aus der Gruppe bestehend aus Calciumdihydrogenphosphat, Calciumhydrogenphosphat, Calciumphosphat, Hydroxyapatit, Brushit und Kombinationen hiervon, enthält oder daraus besteht, wobei das Calciumphosphat bevorzugt Hydroxyapatit und/oder Brushit enthält oder daraus besteht.

11. Verfahren gemäß einem der Ansprüche 9 oder 10, **dadurch gekennzeichnet, dass** ein Grundkörper bereitgestellt wird, der ein Material, das ausgewählt ist aus der Gruppe bestehend aus Metallen, Halbmetallen, Kohlenstoffen, Kunststoffen und Keramiken, bevorzugt ausgewählt ist aus der Gruppe der Metalle, besonders bevorzugt ausgewählt ist aus der Gruppe der Leichtmetalle, insbesondere Titan oder eine Titanlegierung, enthält oder daraus besteht.

12. Verfahren gemäß einem der Ansprüche 9 bis 11, **dadurch gekennzeichnet, dass** ein Grundkörper bereitgestellt wird, dessen elektrisch leitfähiges Material
i) auf den Grundkörper aufgebracht ist oder einstückig mit dem Grundkörper ist; und/oder
ii) ausgewählt ist aus der Gruppe bestehend aus Metallen, Halbmetallen, Kohlenstoffen und Kunststoffen, bevorzugt ausgewählt ist aus der Gruppe der Metalle, besonders bevorzugt, ausgewählt ist aus der Gruppe bestehend aus Titan, Titanlegierung, Tantal, Magnesium und Legierungen hiervon.

13. Verfahren gemäß einem der Ansprüche 9 bis 12, **dadurch gekennzeichnet, dass** auf dem elektrisch leitfähigen Material des Grundkörpers eine Calciumphosphat-Schicht aufgebracht wird, die Calciumphosphat enthält oder daraus besteht, wobei die Calciumphosphat-Schicht Calciumphosphat bevorzugt in einem Gewichtsprozent-Anteil enthält, der höher ist als ein Gewichtsprozentanteil an Calciumhydroxid in dieser Schicht, wobei sich der Gewichtsprozent-Anteil auf das Gesamtgewicht der Calciumphosphat-Schicht bezieht, wobei die Calciumphosphat-Schicht bevorzugt so aufgebracht wird, das sie das elektrisch leitfähige Material des Grundkörpers kontaktiert und/oder die Calciumhydroxid-Schicht kontaktiert, wobei die Calciumphosphat-Schicht insbesondere
i) über eine elektrochemische Abscheidung oder einen Plasmaspritzprozess auf dem elektrisch leitfähigen Material des Grundkörpers abgeschieden wird; und/oder
ii) als Schicht aufgebracht wird, die eine gleiche Schichtdicke über die gesamte Ausdehnung der Schicht aufweist; und/oder
iii) als poröse Schicht aufgebracht wird; und/oder
iv) bis zu einer Schichtdicke im Bereich von 2 bis 500 µm, bevorzugt im Bereich von 5 bis 200 µm, besonders bevorzugt im Bereich von 10 bis 130 µm, aufgebracht wird.

14. Verfahren gemäß einem der Ansprüche 9 bis 13, **dadurch gekennzeichnet, dass** die Calciumhydroxid-Schicht
i) über eine elektrochemische Abscheidung abgeschieden ist, bevorzugt über eine elektrochemische Abscheidung mit einem Elektrolyten, der Calciumnitrat, Ammoniumdihydrogenphosphat und Citronensäure enthält und besonders bevorzugt einen pH Wert von < 7 aufweist; und/oder
ii) als Schicht aufgebracht wird, die eine gleiche Schichtdicke über die gesamte Ausdehnung der Schicht aufweist; und/oder
iii) bis zu einer Schichtdicke im Bereich von 1 bis 50 µm, bevorzugt 2 bis 40 µm, besonders bevorzugt >20 bis 30 µm, aufgebracht wird.

15. Verfahren gemäß einem der Ansprüche 9 bis 14, **dadurch gekennzeichnet, dass** der Anteil an Calciumphosphat im Bereich von > 0 Gew.-% bis < 40 Gew.-%, bevorzugt von >2 Gew.-% und <20 Gew.-%, bezogen auf das Gesamtgewicht der Calciumhydroxid-Schicht, liegt, wobei das Calciumphosphat besonders bevorzugt in Form von Partikeln in der Calciumhydroxid-Schicht vorliegt, insbesondere in Form von Partikeln mit einer Partikelgröße im Bereich von < 1 µm.

## Claims

1. An implant or implant component comprising
a) a main body of an implant or of an implant component, said main body comprising or consisting of an electrically conductive material at least at a surface; and
b) a calcium hydroxide layer which comprises or consists of calcium hydroxide, the calcium hydroxide layer being applied onto the electrically conductive material of the main body;
**characterized in that** the calcium hydroxide layer comprises calcium phosphate in a percent by weight proportion that is lower than the percent by weight proportion of calcium hydroxide, the percent by weight proportion relating to the total weight of the calcium hydroxide layer.

2. The implant or implant component according to the preceding claim, **characterized in that** the calcium phosphate contained in the calcium hydroxide layer comprises or consists of a calcium phosphate that is selected from the group consisting of calcium dihydrogen phosphate, calcium hydrogen phosphate, calcium phosphate, hydroxyapatite, brushite and combinations hereof, the calcium phosphate contained in the calcium hydroxide layer preferably comprising or consisting of hydroxyapatite and/or brushite.

3. The implant or implant component according to any one of the preceding claims, **characterized in that** the main body comprises or consists of a material that is selected from the group consisting of metals, metalloids, carbons, plastics and ceramics, preferably selected from the group of metals, particularly preferably selected from the group of light metals, in particular titanium or a titanium alloy.

4. The implant or implant component according to any one of the preceding claims, **characterized in that** the electrically conductive material of the main body
i) is applied onto the main body or is formed integrally with the main body; and/or
ii) is selected from the group consisting of metals, metalloids, carbons and plastics, preferably is selected from the group of metals, particularly preferably is selected from the group consisting of titanium, titanium alloy, tantalum, magnesium and alloys thereof.

5. The implant or implant component according to any one of the preceding claims, **characterized in that** a calcium phosphate layer comprising or consisting of calcium phosphate is applied onto the electrically conductive material of the main body, the calcium phosphate layer preferably comprising calcium phosphate in a percent by weight proportion that is higher than a percent by weight proportion of calcium hydroxide in this layer, the percent by weight proportion relating to the total weight of the calcium phosphate layer, the calcium phosphate layer preferably contacting the electrically conductive material of the main body and/or contacting the calcium hydroxide layer, the calcium phosphate layer particularly
i) being deposited on the electrically conductive material of the main body by means of an electrochemical deposition or a plasma spraying process; and/or
ii) being present in the form of a layer that has a constant layer thickness over the entire extent of the layer; and/or
iii) being present in the form of a porous layer; and/or
iv) having a layer thickness in the range of from 2 to 500 µm, preferably in the range of from 5 to 200 µm, particularly preferably in the range of from 10 to 130 µm.

6. The implant or implant component according to any one of the preceding claims, **characterized in that** the calcium hydroxide layer
i) is deposited by means of an electrochemical deposition, preferably by means of an electrochemical deposition with an electrolyte comprising calcium nitrate, ammonium dihydrogen phosphate and citric acid, and particularly preferably having a pH value of < 7; and/or
ii) is present in the form of a layer that has a constant layer thickness over the entire extent of the layer; and/or
iii) has a layer thickness in the range of from 1 to 50 µm, preferably 2 to 40 µm, particularly preferably > 20 to 30 µm.

7. The implant or implant component according to any one of the preceding claims, **characterized in that** the proportion of calcium phosphate in the calcium hydroxide layer lies in the range of > 0 wt.% and < 40 wt.%, preferably of > 2 wt.% and < 20 wt.%, in relation to the total weight of the calcium hydroxide layer, the calcium phosphate being present particularly preferably in the form of particles in the calcium hydroxide layer, in particular in the form of particles with a particle size of < 1 µm.

8. The implant or implant component according to any one of the preceding claims for use in medicine, preferably for use in surgery.

9. A method for producing an implant or an implant component, said method comprising the steps of
a) providing a main body of an implant or of an implant component, the main body comprising or consisting of an electrically conductive material at least at a surface; and
b) applying a calcium hydroxide layer, which comprises or consists of calcium hydroxide, onto the electrically conductive material of the main body;
**characterized in that** the calcium hydroxide layer is applied so that it comprises calcium phosphate in a percent by weight proportion that is lower than the percent by weight proportion of calcium hydroxide, the percent by weight proportion relating to the total weight of the calcium hydroxide layer.

10. The method according to claim 9, **characterized in that** the calcium phosphate contained in the calcium hydroxide layer comprises or consists of a calcium phosphate that is selected from the group consisting of calcium dihydrogen phosphate, calcium hydrogen phosphate, calcium phosphate, hydroxyapatite, brushite and combinations hereof, the calcium phosphate preferably comprising or consisting of hydroxyapatite and/or brushite.

11. The method according to any one of claims 9 or 10, **characterized in that** a main body is provided that comprises or consists of a material that is selected from the group consisting of metals, metalloids, carbons, plastics and ceramics, preferably is selected from the group of metals, particularly preferably is selected from the group of light metals, in particular titanium or a titanium alloy.

12. The method according to any one of claims 9 to 11, **characterized in that** a main body is provided, the electrically conductive material of which
i) is applied onto the main body or is formed integrally with the main body; and/or
ii) is selected from the group consisting of metals, metalloids, carbons and plastics, preferably is selected from the group of metals, particularly preferably is selected from the group consisting of titanium, titanium alloy, tantalum, magnesium and alloys thereof.

13. The method according to any one of claims 9 to 12, **characterized in that** a calcium phosphate layer comprising or consisting of calcium phosphate is applied onto the electrically conductive material of the main body, the calcium phosphate layer preferably comprising calcium phosphate in a percent by weight proportion that is higher than a percent by weight proportion of calcium hydroxide in this layer, the percent by weight proportion relating to the total weight of the calcium phosphate layer, the calcium phosphate layer preferably being applied so that it contacts the electrically conductive material of the main body and/or contacts the calcium hydroxide layer, the calcium phosphate layer particularly
i) being deposited on the electrically conductive material of the main body by means of an electrochemical deposition or a plasma spraying process; and/or
ii) being applied in the form of a layer that has a constant layer thickness over the entire extent of the layer; and/or
iii) being applied in the form of a porous layer; and/or
iv) being applied up to a layer thickness in the range of from 2 to 500 µm, preferably in the range of from 5 to 200 µm, particularly preferably in the range of from 10 to 130 µm.

14. The method according to any one of claims 9 to 13, **characterized in that** the calcium hydroxide layer
i) is deposited by means of an electrochemical deposition, preferably by means of an electrochemical deposition with an electrolyte comprising calcium nitrate, ammonium dihydrogen phosphate and citric acid, and particularly preferably having a pH value of < 7; and/or
ii) is applied in the form of a layer that has a constant layer thickness over the entire extent of the layer; and/or
iii) is applied up to a layer thickness in the range of from 1 to 50 µm, preferably 2 to 40 µm, particularly preferably > 20 to 30 µm.

15. The method according to any one of claims 9 to 14, **characterized in that** the proportion of calcium phosphate lies in the range of from > 0 wt.% to < 40 wt.%, preferably from > 2 wt.% and < 20 wt.%, in relation to the total weight of the calcium hydroxide layer, the calcium phosphate being present particularly preferably in the form of particles in the calcium hydroxide layer, in particular in the form of particles with a particle size in the range of < 1 µm.

## Revendications

1. Implant ou composant d'implant, contenant
a) un corps de base d'un implant ou d'un composant d'implant, contenant un matériau électriquement conducteur, ou constitué dudit matériau, sur au moins une surface ; et
b) une couche d'hydroxyde de calcium contenant de l'hydroxyde de calcium ou constituée d'hydroxyde de calcium, dans lequel la couche d'hydroxyde de calcium est appliquée sur le matériau électriquement conducteur du corps de base ;
**caractérisé en ce que** la couche d'hydroxyde de calcium contient du phosphate de calcium dans une proportion en poids qui est inférieure à la proportion en poids d'hydroxyde de calcium, dans lequel la proportion en poids se rapporte au poids total de la couche d'hydroxyde de calcium.

2. Implant ou composant d'implant selon la revendication précédente, **caractérisé en ce que** le phosphate de calcium contenu dans la couche d'hydroxyde de calcium contient un, ou est constitué d'un, phosphate de calcium choisi dans le groupe constitué par le dihydrogénophosphate de calcium, l'hydrogénophosphate de calcium, le phosphate de calcium, l'hydroxyapatite, la brushite et des combinaisons de ceux-ci, dans lequel le phosphate de calcium contenu dans la couche d'hydroxyde de calcium contient de manière préférée de l'hydroxyapatite et/ou de la brushite ou en est constitué.

3. Implant ou composant d'implant selon l'une quelconque des revendications précédentes, **caractérisé en ce que** le corps de base contient un matériau qui est choisi dans le groupe constitué par les métaux, les demi-métaux, les carbones, les plastiques et les céramiques, de manière préférée est choisi dans le groupe des métaux, de manière particulièrement préférée est choisi dans le groupe des métaux légers, en particulier le titane ou un alliage de titane.

4. Implant ou composant d'implant selon l'une quelconque des revendications précédentes, **caractérisé en ce que** le matériau électriquement conducteur du corps de base
i) est appliqué sur le corps de base ou fait partie intégrante du corps de base ; et/ou
ii) est choisi dans le groupe constitué par les métaux, les demi-métaux, les carbones et les plastiques, est choisi de manière préférée dans le groupe des métaux, est choisi de manière particulièrement préférée dans le groupe constitué par le titane, l'alliage de titane, le tantale, le magnésium et les alliages de ceux-ci.

5. Implant ou composant d'implant selon l'une quelconque des revendications précédentes, **caractérisé en ce qu'**une couche de phosphate de calcium contenant du phosphate de calcium ou constituée de phosphate de calcium est appliquée sur le matériau électriquement conducteur du corps de base, dans lequel la couche de phosphate de calcium contient de manière préférée du phosphate de calcium dans une proportion en poids supérieure à une proportion en poids d'hydroxyde de calcium dans ladite couche, dans lequel la proportion en poids se rapporte au poids total de la couche de phosphate de calcium, dans lequel la couche de phosphate de calcium est en contact de manière préférée avec le matériau électriquement conducteur du corps de base et/ou est en contact avec la couche d'hydroxyde de calcium, dans lequel la couche de phosphate de calcium en particulier
i) est déposée sur le matériau électriquement conducteur du corps de base par l'intermédiaire d'un dépôt électrochimique ou d'un processus de pulvérisation plasma ; et/ou
ii) se présente sous la forme d'une couche présentant une épaisseur de couche égale sur toute l'étendue de la couche ; et/ou
iii) se présente sous la forme d'une couche poreuse ; et/ou
iv) présente une épaisseur de couche dans la plage comprise entre 2 et 500 µm, de manière préférée dans la plage comprise entre 5 et 200 µm, de manière particulièrement préférée dans la plage comprise entre 10 et 130 µm.

6. Implant ou composant d'implant selon l'une quelconque des revendications précédentes, **caractérisé en ce que** la couche d'hydroxyde de calcium
i) est déposée par l'intermédiaire d'un dépôt électrochimique, de manière préférée par l'intermédiaire d'un dépôt électrochimique avec un électrolyte contenant du nitrate de calcium, du dihydrogénophosphate d'ammonium et de l'acide citrique et présentant de manière particulièrement préférée une valeur de pH <7 ; et/ou
ii) se présente sous la forme d'une couche présentant une épaisseur de couche égale sur toute l'étendue de la couche ; et/ou
iii) présente une épaisseur de couche dans la plage comprise entre 1 et 50 µm, de manière préférée comprise entre 2 et 40 µm, de manière particulièrement préférée comprise entre >20 et 30 µm.

7. Implant ou composant d'implant selon l'une quelconque des revendications précédentes, **caractérisé en ce que** la proportion de phosphate de calcium dans la couche d'hydroxyde de calcium se situe dans la plage comprise entre >0 % en poids et <40 % en poids, de manière préférée entre >2 % en poids et <20 % en poids, par rapport au poids total de la couche d'hydroxyde de calcium, dans lequel le phosphate de calcium se présente de manière particulièrement préférée sous la forme de particules dans la couche d'hydroxyde de calcium, en particulier sous la forme de particules présentant une taille de particules <1 µm.

8. Implant ou composant d'implant selon l'une quelconque des revendications précédentes pour une utilisation en médecine, de manière préférée pour une utilisation en chirurgie.

9. Procédé de fabrication d'un implant ou d'un composant d'implant, comprenant les étapes consistant à :
a) fournir un corps de base d'un implant ou d'un composant d'implant, dans lequel le corps de base contient un matériau électriquement conducteur, ou est constitué d'un matériau électriquement conducteur, au moins sur une surface ; et
b) appliquer sur le matériau électriquement conducteur du corps de base une couche d'hydroxyde de calcium contenant de l'hydroxyde de calcium ou constituée d'hydroxyde de calcium ;
**caractérisé en ce que** la couche d'hydroxyde de calcium est appliquée de manière à contenir du phosphate de calcium dans une proportion en poids qui est inférieure à la proportion en poids d'hydroxyde de calcium, dans lequel la proportion en poids se rapporte au poids total de la couche d'hydroxyde de calcium.

10. Procédé selon la revendication 9, **caractérisé en ce que** le phosphate de calcium contenu dans la couche d'hydroxyde de calcium contient un, ou est constitué d'un, phosphate de calcium choisi dans le groupe constitué par le dihydrogénophosphate de calcium, l'hydrogénophosphate de calcium, le phosphate de calcium, l'hydroxyapatite, la brushite et des combinaisons de ceux-ci, dans lequel le phosphate de calcium contient de manière préférée de l'hydroxyapatite et/ou de la brushite ou en est constitué.

11. Procédé selon l'une quelconque des revendications 9 ou 10, **caractérisé en ce qu'**un corps de base est fourni, qui contient un, ou est constitué d'un, matériau choisi dans le groupe constitué par les métaux, les demi-métaux, les carbones, les plastiques et les céramiques, de manière préférée choisi dans le groupe des métaux, de manière particulièrement préférée choisi dans le groupe des métaux légers, en particulier le titane ou un alliage de titane.

12. Procédé selon l'une quelconque des revendications 9 à 11, **caractérisé en ce qu'**un corps de base est fourni, dont le matériau électriquement conducteur
i) est appliqué sur le corps de base ou fait partie intégrante du corps de base ; et/ou
ii) est choisi dans le groupe constitué par les métaux, les demi-métaux, les carbones et les plastiques, de manière préférée est choisi dans le groupe des métaux, de manière particulièrement préférée est choisi dans le groupe constitué par le titane, l'alliage de titane, le tantale, le magnésium et les alliages de ceux-ci.

13. Procédé selon l'une quelconque des revendications 9 à 12, **caractérisé en ce qu'**une couche de phosphate de calcium contenant du phosphate de calcium ou constituée de phosphate de calcium est appliquée sur le matériau électriquement conducteur du corps de base, dans lequel la couche de phosphate de calcium contient de manière préférée du phosphate de calcium dans une proportion en poids qui est supérieure à une proportion en poids d'hydroxyde de calcium dans ladite couche, dans lequel la proportion en poids se rapporte au poids total de la couche de phosphate de calcium, dans lequel la couche de phosphate de calcium est de manière préférée appliquée de manière à être en contact avec le matériau électriquement conducteur du corps de base et/ou à être en contact avec la couche d'hydroxyde de calcium, dans lequel la couche de phosphate de calcium en particulier
i) est déposée sur le matériau électriquement conducteur du corps de base par l'intermédiaire d'un dépôt électrochimique ou d'un procédé de pulvérisation plasma ; et/ou
ii) est appliquée sous la forme d'une couche présentant une épaisseur de couche égale sur toute l'étendue de la couche ; et/ou
iii) est appliquée sous la forme d'une couche poreuse ; et/ou
iv) est appliquée jusqu'à une épaisseur de couche dans la plage comprise entre 2 et 500 µm, de manière préférée dans la plage comprise entre 5 et 200 µm, de manière particulièrement préférée dans la plage compris entre 10 et 130 µm.

14. Procédé selon l'une quelconque des revendications 9 à 13, **caractérisé en ce que** la couche d'hydroxyde de calcium
i) est déposée par l'intermédiaire d'un dépôt électrochimique, de manière préférée par l'intermédiaire d'un dépôt électrochimique avec un électrolyte contenant du nitrate de calcium, du dihydrogénophosphate d'ammonium et de l'acide citrique et présentant de manière particulièrement préférée une valeur de pH <7 ; et/ou
ii) est appliquée sous la forme d'une couche présentant une épaisseur de couche égale sur toute l'étendue de la couche ; et/ou
iii) est appliquée jusqu'à une épaisseur de couche dans la plage comprise entre 1 et 50 µm, de manière préférée comprise entre 2 et 40 µm, de manière particulièrement préférée comprise entre >20 et 30 µm.

15. Procédé selon l'une quelconque des revendications 9 à 14, **caractérisé en ce que** la proportion de phosphate de calcium se situe dans la plage comprise entre >0 % en poids et <40 % en poids, de manière préférée comprise entre >2 % en poids et <20 % en poids, par rapport au poids total de la couche d'hydroxyde de calcium, dans lequel le phosphate de calcium se présente de manière particulièrement préférée sous la forme de particules dans la couche d'hydroxyde de calcium, en particulier sous la forme de particules présentant une taille de particules dans la plage <1 µm.
